# EUROPEAN PATENT SPECIFICATION

(11) **EP 2 540 280 B1**
(45) Date of publication and mention of the grant of the patent: **10.06.2015**
(21) Application number: 11718761.7
(22) Date of filing: 25.02.2011
(51) Int. Cl.: A61K 36/74, A61K 8/97, A61Q 19/00

(54) **ASH OF COFFEE HUSKS AFTER ROASTING AND SEPARATION FROM THE COFFEE KERNEL FOR USE AS TOPICAL MEDICINE**
ASCHE VON KAFFEE HUELSEN NACH BRENNUNG UND TRENNUNG VON DEM KAFFEE-KERN ZUR VERWENDUNG ALS TOPISCHES ARZNEIMITTEL
CENDRES DE PARCHES DE CAFÉ APRÈS LEUR TORRÉFACTION ET LEUR SÉPARATION DU GRAIN DE CAFÉ UTILISÉES EN TANT QUE MÉDICAMENT TOPIQUE

(30) Priority: 26.02.2010 ES 201030290
(43) Date of publication of application: 02.01.2013
(73) Proprietor: Pont González, Feliu, 08206 Sabadell (ES)
(72) Inventor: Pont González, Feliu, 08206 Sabadell (ES)
(74) Representative: Manresa Val, Manuel
(86) International application number: PCT/ES2011/070126
(87) International publication number: WO 2011/104415

(56) References cited:
- WO-A1-03/000810
- WO-A2-2005/102373
- BE-A1- 903 646
- US-A1- 2009 142 412

## Description

Ash from coffee husks after being roasted and separated from the coffee core for use as skin medication.

Ash from coffee husks after being roasted and separated from the coffee core for use in the treatment of skin ailments.

### BACKGROUND TO THE INVENTION

The use of unroasted coffee is known in the treatment of psoriasis.

The use of said coffee, in natural form, is known, taking advantage of the acids released by the coffee core.

Belgian patent 903646, "COMPOSITION POUR LE TRAITEMENT ET L'ENTRETIEN DE LA PEAU" is known from 1985, in the name of Mr Goy A. Huysmans, which refers to a composition for skin care and treatment. It comprises a suspension or solution of caffeine (i) or a purine-type alkaloid with a pharmacologic acid (ii). Preferably the powdered and alkaloid vegetable material compound, for example, coffee, tea, cacao or cola, especially finely ground unroasted coffee in a proportion of 20/80 (25-45)% by composition weight. The acid is preferably salicylic, fumaric, citric, malic, tartaric or oxalic acid, especially between 5-25 (5-15)% in weight of salicylic or fumaric acid.

### BRIEF DISCLOSURE OF THE INVENTION

This invention deals with the use of roasted coffee husks as a skin ailment efficient treatment.

There is currently no effective remedy for skin ailments because each one has its own treatment. The closest document is Belgian patent 903646. This describes how raw coffee beans are powdered and then mixed with an acid.

This invention employs the coffee husks after they are roasted. Said husks are converted into ash and usually are in powder form. They have a high content in potassium, calcium, magnesium, sulphur and phosphorous.

Therefore, compared with the closest prior art document, roasted coffee husks are used instead of unroasted and, in addition, they are not mixed with any acid and are directly applied to the skin. It is an initially dry product, without any organic material.

Roasted coffee husks are a moisture-free product. In skin ailments, such as psoriasis, the patient worsens with moisture because said moisture impedes scar tissue formation so that the use of these husks favours psoriasis treatment because it dries the skin.

The mentioned husks contain sulphur, which is known to be an effective means of combating psoriasis, such as, sulphur water baths, which encourage scar tissue formation, with the advantage of not moisturising the treated area.

As is well known, coffee contains antioxidants that are also an effective psoriasis treatment.

This means that said husks combine various elements that combat skin ailments, with the advantage that they come from a substance that was previously discarded - roasted coffee husks.

Moreover, analysis of said ash has not revealed any organic products. This is significant in that ulceration may be present which is not aggravated by the ash since it does not cause any infection.

One object of this invention is ash from coffee husks after being roasted and separated from the coffee core for use as topical medication.

An additional object of this invention is ash from coffee husks after being roasted and separated from the coffee core for use in skin ailment treatment.

### SPECIFIC EMBODIMENT OF THE INVENTION

As previously described, the roasted coffee husks, which forms part of the discarded coffee, are employed.

Because of the roasting process, said husks mainly appear in powder form without any moisture due to the coffee roasting temperatures.

It is subsequently collected and, when cool, is applied to the skin and gently massaged into it.

Depending on the degree of psoriatic dermatitis, improvements have been noted within one week.

In more serious cases, tests have shown that an average of one month leads to curing.

In patients with ulceration, ash-based treatment is extended up to two months.

Lastly, cases of lichen planus require an average period of two months.

Studies are currently being performed with diseases, such as leprosy, with encouraging results.

Although it was initially intended to employ this coffee husks powder without any cream or other support vehicle, on occasions, it may have to be used with other compounds, for example, mixed with aloe vera, in creams or solutions. In certain circumstances, effectiveness is increased because the cream or solution vehicle aids skin penetration.

A combination of iodine with the roasted coffee husks powder has been found to be especially useful in cases in which infections become in ulcerations.

Analysis of said roasted coffee husks powder shows a high content in potassium, calcium, magnesium, sulphur and phosphorous, together with smaller amounts of copper and zinc.

It has also been observed that with low caffeine levels, the roasted coffee husks powder is less abrasive.

The use of natural roasted coffee from any source is recommended because it is the purest.

One way to use the ash is after showering. On the one hand, it helps to dry the affected zone and, on the other, it can employ the available moisture for absorbing the metal content.

It is therefore claimed that the coffee husks ash after roasting and separation from the core, be employed as skin medication, with a specific use in skin ailment treatment.

This invention describes the use of coffee husks ash after roasting and separation from the core as skin medication and the use of coffee husks ash after roasting and separation from the core for skin ailment treatment. The examples mentioned here do not limit this invention and may therefore have various applications and/or adaptations, all of which are within the scope of the following claims.

## Claims

1. Coffee husks ash after roasting and separation from the core for use as topical medication.

2. Ash in accordance with claim 1 **characterised in that** the husks are in powder form.

3. Ash in accordance with either of the previous claims **characterised in that** it contains potassium, calcium, magnesium, sulphur and phosphorous.

4. Ash in accordance with any of the previous claims **characterised in that** it is mixed with iodine.

5. Ash in accordance with any of the previous claims **characterised in that** the coffee is natural.

6. Coffee husks ash after roasting and separation from the core for use in skin ailment treatment.

7. Ash in accordance with claim 6, **characterised in that** the husks are in powder form.

8. Ash in accordance with either claim 6 or 7 **characterised in that** it contains potassium, calcium, magnesium, sulphur and phosphorous.

9. Ash in accordance with any of the previous claims 6 to 8 **characterised in that** it is mixed with iodine.

10. Ash in accordance with any of the previous claims 6 to 9 **characterised in that** the coffee is natural.

## Patentansprüche

1. Asche aus Kaffeekirschenschalen nach dem Rösten und Trennung vom Kern zur Verwendung als topisches Medikament.

2. Asche gemäß Anspruch 1, **dadurch gekennzeichnet, dass** die Schalen in Pulverform sind.

3. Asche gemäß einem der vorherigen Ansprüche, **dadurch gekennzeichnet, dass** sie Kalium, Kalzium, Magnesium, Schwefel und Phosphor enthält.

4. Asche gemäß einem der vorherigen Ansprüche, **dadurch gekennzeichnet, dass** sie mit Jod gemischt ist.

5. Asche gemäß einem der vorherigen Ansprüche, **dadurch gekennzeichnet, dass** es sich um natürlichen Kaffee handelt.

6. Asche aus Kaffeekirschenschalen nach dem Rösten und Trennung vom Kern zur Verwendung bei der Behandlung von Hautkrankheiten.

7. Asche gemäß Anspruch 6, **dadurch gekennzeichnet, dass** die Schalen in Pulverform sind.

8. Asche gemäß dem Anspruch 6 oder 7, **dadurch gekennzeichnet, dass** sie Kalium, Kalzium, Magnesium, Schwefel und Phosphor enthält.

9. Asche gemäß einem der vorherigen Ansprüche 6 bis 8, **dadurch gekennzeichnet, dass** sie mit Jod gemischt ist.

10. Asche gemäß einem der vorherigen Ansprüche 6 bis 9, **dadurch gekennzeichnet, dass** es sich um natürlichen Kaffee handelt.

## Revendications

1. Cendre de parches de café après torréfaction et séparation du grain, pour son utilisation comme médicament topique.

2. Cendre conformément à la revendication 1, **caractérisée en ce que** les parches sont sous forme de poudre.

3. Cendre conformément à l'une des deux revendications précédentes, **caractérisée en ce qu'**elle contient du potassium, du calcium, du magnésium, du soufre et du phosphore.

4. Cendre conformément à l'une quelconque des revendications précédentes, **caractérisée en ce qu'**elle est mélangée avec de l'iode.

5. Cendre conformément à l'une quelconque des revendications précédentes, **caractérisée en ce que** le café est naturel.

6. Cendre de parches de café après torréfaction et séparation du grain pour son utilisation comme traitement de maladie de la peau.

7. Cendre conformément à la revendication 6, **caractérisée en ce que** les parches sont sous forme de poudre.

8. Cendre conformément à l'une des revendications 6 ou 7, **caractérisée en ce qu'**elle contient du potassium, du calcium, du magnésium, du soufre et du phosphore.

9. Cendre conformément à l'une quelconque des revendications 6 à 8 précédentes, **caractérisée en ce qu'**elle est mélangée avec de l'iode.

10. Cendre conformément à l'une quelconque des revendications 6 à 9 précédentes, **caractérisée en ce que** le café est naturel.
